# EUROPEAN PATENT SPECIFICATION

(11) **EP 2 366 361 B1**
(45) Date of publication and mention of the grant of the patent: **06.09.2017**
(21) Application number: 11001866.0
(22) Date of filing: 07.03.2011
(51) Int. Cl.: A61C 19/04, A61B 5/053, A61B 5/00, A61B 5/107

(54) **Treatment unit**
Behandlungseinheit
Unité de traitement

(30) Priority: 16.03.2010 JP 2010058711
(43) Date of publication of application: 21.09.2011
(73) Proprietor: J. Morita Manufacturing Corporation, Kyoto-shi, Kyoto 612-8533 (JP)
(72) Inventor: Yamashita, Seiichiro, Kyoto-shi Kyoto 612-8533 (JP); Kusakabe, Hiroaki, Kyoto-shi Kyoto 612-8533 (JP); Matoba, Kazunari, Kyoto-shi Kyoto 612-8533 (JP)
(74) Representative: Müller Hoffmann & Partner

(56) References cited:
- DE-A1- 10 219 648
- DE-A1- 19 628 854
- JP-A- 8 000 640

## Description

### BACKGROUND OF THE INVENTION

### Field of the Invention

The present invention relates to a treatment unit used in the field of healthcare including medical and dental fields. The present invention specifically relates to a technique intended for size reduction and weight reduction of a treatment unit.

### Description of the Background Art

According to a technique conventionally suggested regarding a treatment unit for medical or dental purposes, a diagnosis is made and curative treatment is given at the same time. As an example, in a suggested root canal treatment unit for dental purposes, a diagnosis such as measurement of a root canal length is made, and curative treatment is given at the same time.

With reference for example to patent literature 1 (Japanese Patent Application Laid-Open No. 8-640 (1996)), it discloses a cordless handpiece for root canal enlargement to which a display unit and a root canal length measuring circuit are provided. The treatment unit disclosed therein includes a large display part on a battery charger on which measured data of a root canal length can be displayed.

### SUMMARY OF THE INVENTION

The aforementioned conventional treatment unit makes it possible to make a diagnosis and give treatment in one treatment unit. However, this in turn results in considerably increased size and complicated structure of the treatment unit. In particular, regarding the treatment unit disclosed in patent literature 1, the handpiece includes a root canal length measuring module as well as a root canal enlarging module, thereby complicating the structure and increasing the size of the handpiece. The considerably increased size of the treatment unit may worsen operability, increase a footprint, or increase the overall weight, by which medical care might be impaired. So, a technique intended for size reduction and weight reduction of a treatment unit has been desired.

The present invention has been made to solve the aforementioned problem. It is an object of the present invention to provide a technique capable of reducing the size and weight of a treatment unit by simplifying the structure of the treatment unit.
This object is solved by the subject-matter of the independent claim 1.

The treatment unit of the present invention entrusts the external diagnostic unit with a diagnostic function the treatment unit itself does not have. Further, the structure of the treatment unit is such that diagnostic information received from the external diagnostic unit is displayed on the display part. This allows simplification of the structure of the treatment unit itself, thereby achieving an advantageous effect as the treatment unit can be reduced in size and weight.

In particular, both the driving information about the treatment unit itself and diagnostic information acquired by the external diagnostic unit can be displayed on the same display part. This achieves an advantageous effect as a user can see the driving information about the treatment unit itself, and can more easily give appropriate treatment based on the diagnostic information.

In particular, the treatment unit body is in the form of a handpiece. This achieves an advantageous effect as a user is allowed to handle the treatment unit body easily.

In particular, the display part is provided on the outer circumference of the holding part. This achieves an advantageous effect as a user of the treatment unit can turn the user's eyes back and forth within a smaller distance between a patient and the display part.

In particular, the treatment unit allows the diagnostic information to be viewed appropriately and easily. More specifically, employing a liquid crystal display or an organic EL display as the display part realizes size reduction while providing a wide variety of display styles. Further, employing an electronic paper as the display part allows the display part to be mounted very easily as the electronic paper is thin, light in weight and easy to bend, especially if the treatment unit is shaped as a handpiece. Still further, employing a light-emitting diode as the display part provides the display device at low cost.

In particular, the treatment unit can be used at a position away from a place from which the diagnostic information is transmitted. This achieves an advantageous effect as treatment is given with a higher degree of flexibility.

In particular, the treatment unit allows treatment based on information about a root canal length while the root canal length is recognized. Information about a root canal length is one of important information in dental treatment. So, this achieves an advantageous effect as it realizes appropriate treatment.

These and other objects, features, aspects and advantages of the present invention will become more apparent from the following detailed description of the present invention when taken in conjunction with the accompanying drawings.

### BRIEF DESCRIPTION OF THE DRAWINGS

Fig. 1 is a perspective view of the outer appearances of a treatment unit and a root canal length measuring unit of a first preferred embodiment;
Fig. 2 is a block diagram of the internal structures of the treatment unit and the root canal length measuring unit;
Fig. 3 is a perspective view of the treatment unit being used;
Figs. 4A and 4B each show a screen on a display device;
Fig. 5 is a perspective view of the outer appearance of a root canal length measuring system;
Fig. 6 is a block diagram of the internal structures of a treatment unit and a root canal length measuring unit of a second preferred embodiment; and
Fig. 7 is a block diagram of the internal structures of a treatment unit and a root canal length measuring unit of a third preferred embodiment.

### DESCRIPTION OF THE PREFERRED EMBODIMENTS

In the following, preferred embodiments of the present invention are described in detail with reference to the accompanying drawings. However, constituent elements shown in the preferred embodiments are merely illustrative, to which the scope of the present invention is not intended to be limited.

### <1. First Preferred Embodiment>

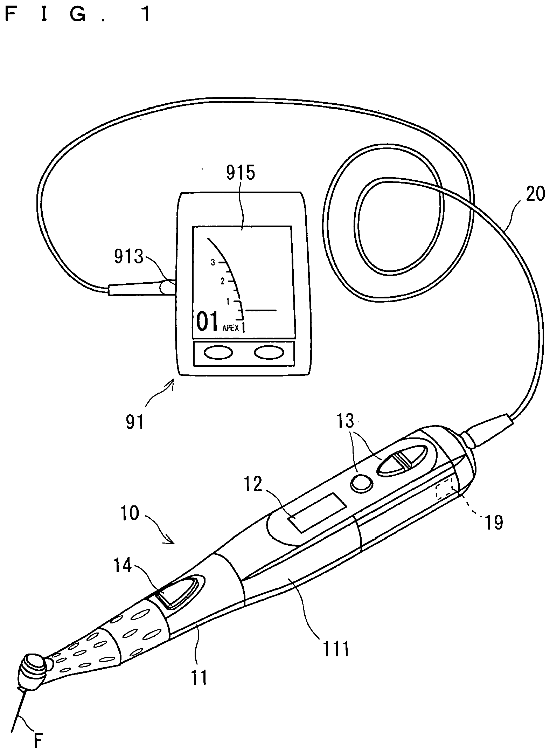

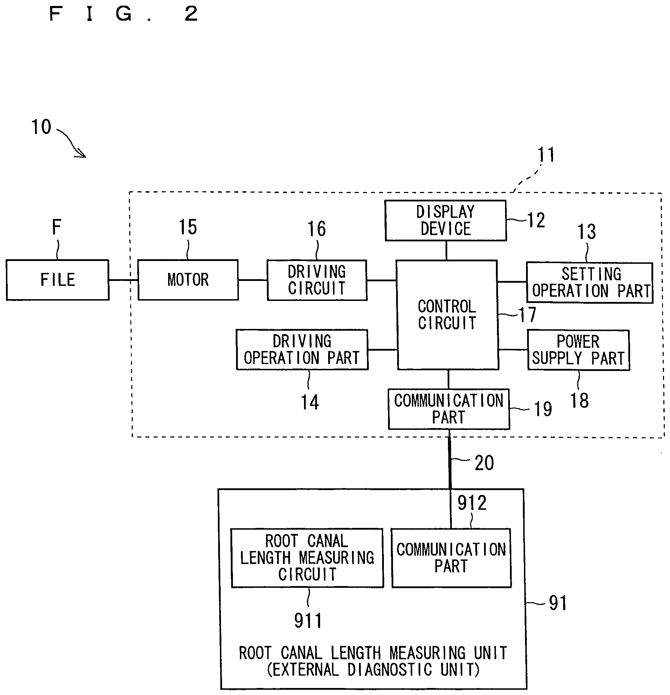

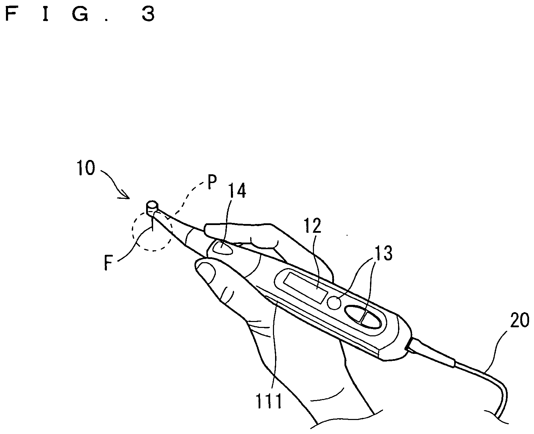

Fig. 1 is a perspective view of the outer appearances of a treatment unit 10 and a root canal length measuring unit 91 of a first preferred embodiment. Fig. 2 is a block diagram of the internal structures of the treatment unit 10 and the root canal length measuring unit 91. Fig. 3 is a perspective view of the treatment unit 10 being used.

As shown in Fig. 1, the treatment unit 10 is a treatment tool substantially in the form of a rod. More specifically, the treatment unit 10 functions as a micro-motor handpiece for root canal enlargement for enlarging a root canal by cutting a tooth with drive of a file F attached to the tip end of the treatment unit 10. The root canal length measuring unit 91 is an external diagnostic unit that is originally intended to be used alone independently of the treatment unit 10. The root canal length measuring unit 91 acquires a root canal length as diagnostic information, and transmits the acquired root canal length to the treatment unit 10. The structures of the treatment unit 10 and the root canal length measuring unit 91 are described in detail below.

As shown in Fig. 1, the treatment unit 10 includes: a treatment unit body 11 that forms a holding part 111 held by a user; a display device 12 that forms a screen on which various types of information are displayed; a setting operation part 13 through which operations of the treatment unit 10 are set; and a driving operation part 14 that controls on and off of the rotation of the file F (treatment tool). As shown in Fig. 2, the treatment unit 10 includes inside the treatment unit 10: a motor 15 for causing the file F to rotate; a driving circuit 16 for causing the motor 15 to rotate; a control circuit 17 for controlling the treatment unit 10 in its entirety; a power supply part 18 for supplying electric power; and a communication part 19 for making communications with an external diagnostic unit.

The display device 12 is provided on the outer circumference of the holding part 111 of the treatment unit body 11 as shown in Fig. 1. The structure of the display device 12 is such that driving information about the motor 15, and information about a root canal length acquired by the root canal length measuring unit 91 are displayed on the display device 12.

The control circuit 17 is constructed of a logic circuit. Based on signals input from the setting operation part 13, the driving operation part 14 and others, the control circuit 17 controls the operations of the driving circuit 16 and the display device 12. More specifically, based on an input made through the setting operation part 13, the control circuit 17 determines which one of driving information and diagnostic information is to be displayed on the display device 12, or determines that both of them are to be displayed on the display device 12. The control circuit 17 also controls the driving circuit 16 based on an input made through the setting operation part 13, so that the number of rotations (rotation speed) and the like of the motor 15 can be determined. The control circuit 17 also controls the driving circuit 16 based on an input made through the driving operation part 14, thereby controlling on and off of the rotation of the motor 15.

The power supply part 18 is constructed of a battery, a power supply circuit, and others, and supplies appropriate electric power to each constituent element of the treatment unit 10 including the driving circuit 16 and the display device 12. In the first preferred embodiment, the power supply part 18 is connected to the control circuit 17. So, the control circuit 17 gives electric power supplied from the power supply part 18 to each constituent element of the treatment unit 10. However, the power supply part 18 may directly supply electric power (namely, without the intervention of a control circuit) to each constituent element of the treatment unit 10.

The power supply source of the power supply part 18 may be an exchangeable disposable primary battery, or may be a rechargeable secondary battery. The power supply part 18 may receive electric power supply from outside through an electrical cord. If a secondary battery is used, the secondary battery may be charged after being taken out off the treatment unit body 11. Or, the secondary battery may be charged by a contact charging system or by a non-contact charging system while being placed in the treatment unit body 11.

The communication part 19 receives diagnostic information under control of the control circuit 17 that is acquired by the root canal length measuring unit 91. The root canal length measuring unit 91 forms a main part in a root canal length measuring system 90 described later for measuring a root canal length. The root canal length measuring unit 91 and the treatment unit 10 are connected through a communication cable 20. The communication cable 20 attached to part of the treatment unit 10 is connected to a communication cable connecting part 913 of the root canal length measuring unit 91. The control circuit 17 sends a request to transmit diagnostic information to the root canal length measuring unit 91 connected to the treatment unit 10 through the communication cable 20. In response to the request, an external diagnostic unit transmits diagnostic information already acquired, and the transmitted information is received by the communication part 19. The diagnostic information transmitted from the root canal length measuring unit 91 is input to the control circuit 17, and is then displayed on the display device 12. However, if the communication cable 20 is connected to the communication cable connecting part 913, the root canal length measuring unit 91 may automatically continue to transmit diagnostic information even if the control circuit 17 does not send a request to transmit diagnostic information to the root canal length measuring unit 91.

The "external diagnostic unit" mentioned above is a unit with one or more diagnostic functions the treatment unit 10 does not have, and which transmits acquired diagnostic information to the communication part 19 of the treatment unit 10. Referring for example to the root canal length measuring unit 91 shown in Figs. 1 and 2, it includes a root canal length measuring circuit 911. So, the root canal length measuring unit 91 is an external diagnostic unit having a root canal length measuring function the treatment unit 10 does not have.

The external diagnostic unit that can be connected to the treatment unit 10 is not limited to the root canal length measuring unit 91. As a matter of course, such an external diagnostic unit may include those having various diagnostic functions such as a measuring unit for measuring the depth of a periodontal pocket, and a fluorescence diagnostic unit for detecting and evaluating fluorescent light emitted from a site of lesion. The treatment unit 10 may be connected to external diagnostic units of different types to obtain diagnostic information from each external diagnostic unit. In this case, identifying information may be transferred between the external diagnostic units and the treatment unit 10 to allow the treatment unit 10 to specify the respective types of the external diagnostic units connected thereto.

As shown in Fig. 3, by placing the display device 12 at an appropriate position while holding the treatment unit 10, a user can visually recognize driving information and information about a root canal length. This allows the user to give a patient P treatment (root canal enlargement) appropriately. The user can also see diagnostic information by turning the user's eyes back and forth between the user's hand and the patient P. This allows the user to continuously give curative treatment while keeping concentration, thereby enhancing efficiency of the curative treatment.

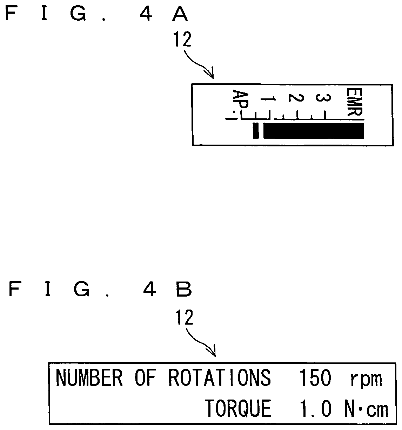

Figs. 4A and 4B each show a screen on the display device 12. Fig. 4A shows diagnostic information displayed on the display device 12, and Fig. 4B shows driving information about the treatment unit 10 itself displayed on the display device 12. As already described, by pressing a button of the setting operation part 13, information to be displayed on a screen can be switched between the diagnostic information shown in Fig. 4A, and the driving information shown in Fig. 4B. As a matter of course, the button of the setting operation part 13 may also be pressed while either diagnostic information or driving information is displayed so that both the diagnostic information and the driving information may be displayed on the screen.

In the first preferred embodiment, information about a root canal length is shown on a meter as shown in Fig. 4A. More specifically, a greater numerical value shown on the meter indicates that the tip end of the file F is farther from an apex of tooth root. Driving information to be displayed includes the number of rotations and the torque of the motor 15 as shown in Fig. 4B. The display style on the display device 12 is not limited to those shown in Figs. 4A and 4B, but of course, may be changed in any way. If constructed of a liquid crystal display, the display device 12 allows diagnostic information to be viewed appropriately and easily.

Driving information and diagnostic information may be displayed together at any time. This allows the diagnostic information and the driving information to be kept track of at the same time during root canal enlargement without requiring operation of the setting operation part 13.

The display device 12 is not limited to a liquid crystal display, but may be constructed of at least one of an organic EL display, an electronic paper, and a light-emitting diode. Employing a liquid crystal display or an organic EL display as the display device 12 realizes size reduction of the display device 12 while providing a wide variety of display styles. Employing an electronic paper as the display device 12 allows the display device 12 to be mounted very easily as the electronic paper is thin, light in weight and easy to bend, especially if the treatment unit 10 is shaped as a handpiece. Employing a light-emitting diode as the display device 12 can easily form the display device 12 at low cost. Only one selected from these display devices may be used. Or, display devices of different types may be used in combination, in which case the display devices may be used separately, or cooperatively according to diagnostic information to be displayed.

### {Root Canal Length Measuring System 90}

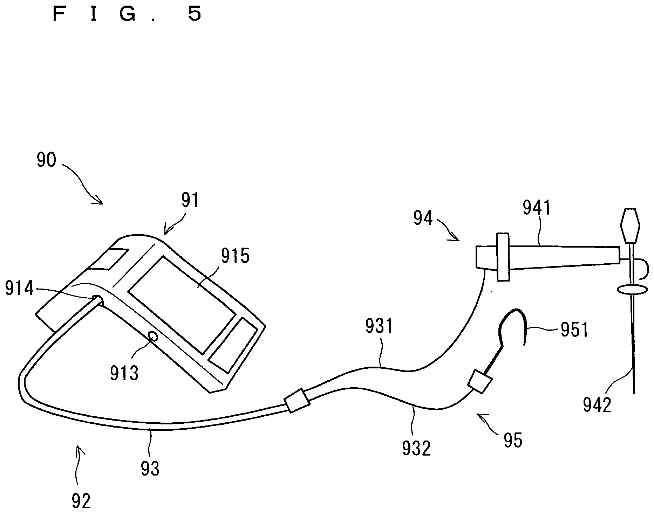

Fig. 5 is a perspective view of the outer appearance of a root canal length measuring system 90. As shown in Fig. 5, the root canal length measuring system 90 includes the root canal length measuring unit 91, a measuring instrument 92, and an electrode cable 93 for connecting the root canal length measuring unit 91 and the measuring instrument 92.

As shown in Fig. 2, the root canal length measuring unit 91 includes a root canal length measuring circuit 911 for measuring a root canal length, and a communication part 912 for making communications with the communication part 19 of the treatment unit 10 that are provided inside the root canal length measuring unit 91. As shown in Fig. 5, the root canal length measuring unit 91 also includes the communication cable connecting part 913, an electrode cable connecting part 914 to which the electrode cable 93 are connected, and a display part 915 on which information about a root canal length is displayed.

The measuring instrument 92 mainly includes positive and negative poles 94 and 95. (Positive and negative poles are differentiated for the convenience of description. However, positive and negative properties of positive and negative poles 94 and 95 always change places if an electrical signal for measuring is an alternating-current signal, which is applied to the present specification.) The positive pole 94 includes a file electrode 941 and a file 942. The electrode cable 93 is constructed of a conductive line 931 connected to the positive pole 94, and a conductive line 932 connected to the negative pole 95 that are provided inside the electrode cable 93. The conductive line 931 extending from an end of the electrode cable 93 is connected to the file electrode 941 of the positive pole 94. The conductive line 932 extending from the end of the electrode cable 93 is connected to an oral electrode 951.

In order to measure a root canal length in the root canal length measuring system 90, the tip end of the file 942 is placed into a root canal of a tooth, and the oral electrode 951 is made to touch the gums or a lip. Then, an electrical measuring signal is applied between the file 942 and the oral electrode 951 in this state to determine the position of the tip end of the root of the tooth (apex). The depth of the root canal is determined by detecting the apex. Various techniques of electrically measuring a root canal length have conventionally been suggested, and which are not explained in detail in the present specification

The structure of the root canal length measuring unit 91 of the first preferred embodiment is such that the communication part 912 transmits acquired information about a root canal length as diagnostic information to the communication part 19 of the treatment unit 10. However, the external diagnostic unit is not necessarily required to transmit diagnostic information directly to the treatment unit 10. The structure of a root canal length measuring unit may also be such that information about a root canal length is transferred between the root canal length measuring unit and a treatment unit with the intervention of an external unit not shown that has a communication function.

As described above, the treatment unit 10 includes the communication part 19 that receives diagnostic information from an external diagnostic unit. So, the treatment unit 10 can entrust the external diagnostic unit with a diagnostic function the treatment unit 10 does not have. Further, the structure of the treatment unit 10 is such that received diagnostic information is displayed on the display device 12. This allows simplification of the structure of the treatment unit 10 itself, thereby achieving size reduction and weight reduction of the treatment unit 10.

As shown in Fig. 3, the display device 12 is provided on the outer circumference of the holding part 111 of the treatment unit 10 shaped as a handpiece. Thus, root canal enlargement is conducted appropriately based on diagnostic information about a root canal length, thereby enhancing treatment accuracy of dental treatment and others.

### Example useful for understanding the invention

In the first preferred embodiment, after the root canal length measuring unit 91 to which the measuring instrument 92 is connected measures a root canal length, the treatment unit 10 is connected to the root canal length measuring unit 91. Then, information about the root canal length is displayed as diagnostic information on the display device 12 of the treatment unit 10. However, this is not the only way of measuring a root canal length with the root canal length measuring unit 91. In the description of the example useful for understanding the invention below, elements having the same functions as those of the first preferred embodiment are designated by the same reference numerals, and are not described repeatedly.

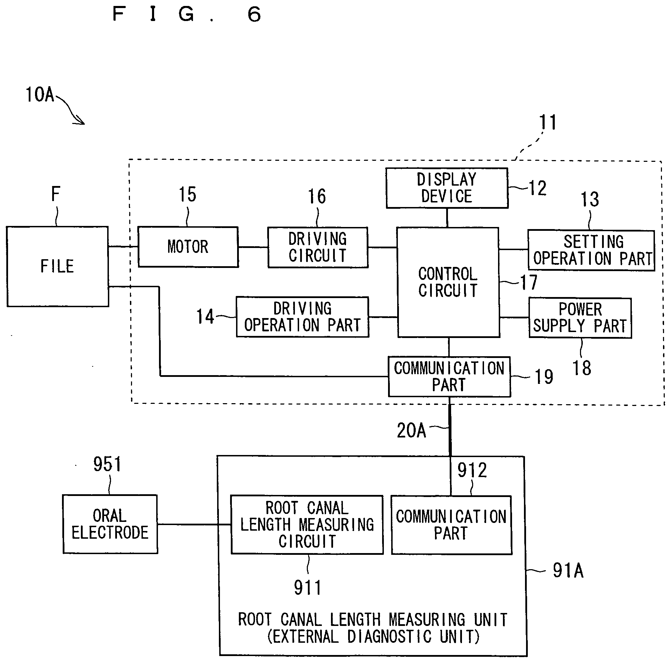

Fig. 6 is a block diagram of the internal structures of a treatment unit 10A and a root canal length measuring unit 91A. A communication cable 20A includes a communication line therein for making communications between the communication part 19 and the communication part 912. This communication line is connected to a conductive line for a positive pole extending from the root canal length measuring circuit 911 of the root canal length measuring unit 91A. This conductive line extends from the communication part 19 through the treatment unit body 11 of the treatment unit 10, and which is then electrically connected to the file F attached to the tip end of the treatment unit 10. The oral electrode 951 is connected through a conductive line for a negative pole to the root canal length measuring circuit 911. Thus, the communication line forming the communication cable 20A functions as part of an electric circuit for root canal length measurement, and the treatment unit 10A functions as the positive pole 94 shown in Fig. 5.

In the aforementioned structures of the treatment unit 10A and the root canal length measuring unit 91A, the root canal length of a targeted tooth is measured by application of an electrical measuring signal between the file F and the oral electrode 951 by the root canal length measuring circuit 911. This means that the treatment unit 10 can be used to measure a root canal length by operating the root canal length measuring unit 91A during root canal enlargement.

The aforementioned communication cable also functions as an electric circuit for an external diagnostic unit. Thus, the treatment unit 10A is capable of reducing the number of components of a medical apparatus. This makes it easier to keep space for the medical apparatus while achieving cost reduction of a treatment unit.

Further, the root canal length measuring circuit 911 can still be put into operation by controlling the root canal length measuring unit 91 by the control circuit 17 through the communication part 19. Operation buttons of the setting operation part 13 and the like provided to the treatment unit body 11 have the function to put the root canal length measuring circuit 911 into operation. Thus, setting for root canal length measurement such as setting of a reference position can be made only by operating the treatment unit 10.

### <Second Preferred Embodiment>

In the preferred embodiments described above, diagnostic information is transferred between an external unit and the treatment unit 10 through the communication cable 20 or 20A. However, a communication system is not limited to that using wired connection.

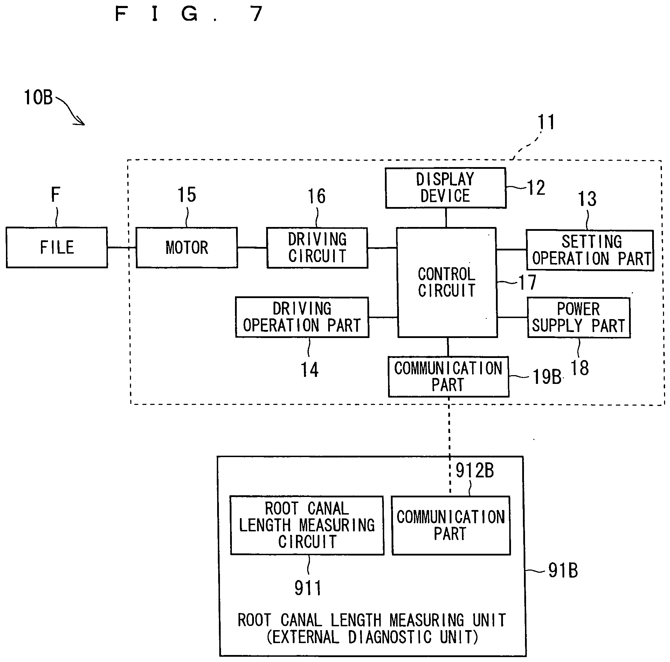

Fig. 7 is a block diagram of the internal structures of a treatment unit 10B and a root canal length measuring unit 91B of a second preferred embodiment. In the second preferred embodiment, the treatment unit 10B includes a communication part 19B, and the root canal length measuring unit 91B includes a communication part 912B. Diagnostic information is transferred by a wireless communication system between the communication parts 19B and 912B. Various communication systems employing wireless communication method including conventional wireless communication techniques may be used for the wireless transfer of diagnostic information.

The second preferred embodiment does not use wired connection, so that spatial restrictions can be relaxed further. Thus, an external diagnostic unit including the root canal length measuring unit 91B and others can be arranged with a higher degree of flexibility, and the operability of the treatment unit 10B as a handpiece is enhanced.

Eliminating physical connection reduces the risk for example of pulling and falling of a cable, while reducing time and labor required for placing a cable and for maintenance.

### <4. Modifications>

The present invention is not limited to the preferred embodiments described above, but various modifications thereof are thinkable.

As an example, the treatment unit 10 in the preferred embodiments has a function to enlarge a root canal, to which the present invention is not limited. The present invention is applicable to various types of treatment units including for example a micro-motor handpiece, a scaler, a photopolymerizing unit, a semiconductor laser and a root canal filling unit that are used for treatment, and a tooth decay detector, a dental pulp tester, an intraoral camera, a periodontal pocket measuring unit, and a shake of tooth measuring unit that are required for medical care. If provided with a communication part for acquiring diagnostic information from an external diagnostic unit, all of these treatment units achieve size reduction and weight reduction of the treatment units.

An external diagnostic unit is not limited to that for measuring a root canal length, but may of course have a different diagnostic function. However, information about a root canal length is one of important information in dental treatment, and is easy to use for controlling the operation of a treatment unit. In this regard, giving curative treatment with information about a root canal length displayed on a display device of a treatment unit enhances efficiency of the curative treatment.

In the preferred embodiments described above, the control circuit 17 is prepared as a dedicated circuit in terms of hardware. The control circuit 17 may alternatively be constructed of a central processing unit (CPU), a ROM in which a program is stored, a temporary memory (such as a RAM), and the like. Namely, the same function as that of the control circuit 17 may be realized in terms of software by causing the CPU to operate on the memory based on the program. Another dedicated circuit may also be realized in terms of software by the central processing unit.

Components of a treatment unit shaped as a handpiece can be separated. Simplifying the internal structure of the handpiece itself realizes weight reduction and size reduction of the handpiece, thereby making it possible for a user to handle the treatment unit easily. As an example, if the display device 12 is placed on a different treatment unit body, diagnostic information can be displayed on a larger screen.

As another example, allowing the communication part 19 or 19B to be retrofitted to the treatment unit 10, 10A or 10B further reduces the weight of the treatment unit body 11 if receipt and display of diagnostic information are not required. In this case, efficiency of curative treatment is enhanced.

While the invention has been shown and described in detail, the foregoing description is in all aspects illustrative and not restrictive. It is therefore understood that numerous modifications and variations can be devised without departing from the scope of the invention as defined in the appended claims.

## Claims

1. A dental treatment unit (10, 10A) comprising:
a communication part (19, 19B) comprising a communication means (20A) adapted to receive a signal indicating a root canal length acquired by an external root canal length measuring unit (91, 91A, 91B) with root canal length measuring function which the treament unit (10, 10A) does not have;
a display part (12) adapted to display the root canal length; and
a treatment unit body (11) used when a dental treatment is given based on the root canal length.

2. The dental treatment unit (10, 10A) according to claim 1,
wherein the display part (12) further displays driving information about the treatment unit itself.

3. The dental treatment unit (10, 10A) according to claim 1 or 2,
wherein the treatment unit body (11) is in the form of a handpiece with a holding part (111) that can be held by a user.

4. The dental treatment unit (10, 10A) according to claim 3,
wherein the display part (12) is provided on the outer circumference of the holding part of the treatment unit body (11).

5. The dental treatment unit (10, 10A) according to any one of claims 1 to 4,
wherein the display part (12) is constructed of at least one of an organic EL display, an electronic paper, and a light-emitting diode.

6. The dental treatment unit (10, 10A) according to any one of claims 1 to 5,
wherein the communication part (19, 19B) receives the signal indicating the root canal length to and from the external root canal length measuring unit (91, 91A, 91B) by a wired communication system through a communication line (20, 20A).

7. The dental treatment unit (10, 10A) according to any one of claims 1 to 5,
wherein the communication part (19, 19B) receives the signal indicating the root canal length by a wireless signal communication system.

## Patentansprüche

1. Zahnbehandlungseinheit (10, 10A), umfassend:
einen Kommunikationsteil (19, 19B), der ein Kommunikationsmittel (20A) umfasst, das dafür geeignet ist, ein Signal zu empfangen, das eine Wurzelkanallänge angibt, die durch eine externe, eine Wurzelkanallänge messende Einheit (91, 91A, 91B) mit einer eine Wurzelkanallänge messenden Funktion, die die Behandlungseinheit (10, 10A) nicht aufweist, erfasst wird;
einen Anzeigeteil (12), der dafür geeignet ist, die Wurzelkanallänge anzuzeigen; und
einen Behandlungseinheitskörper (11), der genutzt wird, wenn basierend auf der Wurzelkanallänge eine Zahnbehandlung durchgeführt wird.

2. Zahnbehandlungseinheit (10, 10A) nach Anspruch 1,
wobei der Anzeigeteil (12) ferner eine Antriebsinformation über die Behandlungseinheit selbst anzeigt.

3. Zahnbehandlungseinheit (10, 10A) nach Anspruch 1 oder 2,
wobei der Behandlungseinheitskörper (11) in der Form eines Handstücks mit einem Halteteil (111) vorliegt, der von einem Nutzer gehalten werden kann.

4. Zahnbehandlungseinheit (10, 10A) nach Anspruch 3,
wobei der Anzeigeteil (12) auf einem äußeren Umfang des Halteteils des Behandlungseinheitskörpers (11) vorgesehen ist.

5. Zahnbehandlungseinheit (10, 10A) nach einem der Ansprüche 1 bis 4,
wobei der Anzeigeteil (12) aus zumindest einem einer organischen EL-Anzeige, eines elektronischen Papiers und einer lichtemittierenden Diode aufgebaut ist.

6. Zahnbehandlungseinheit (10, 10A) nach einem der Ansprüche 1 bis 5,
wobei der Kommunikationsteil (19, 19B) das die Wurzelkanallänge angebende Signal an die und von der externen, eine Wurzelkanallänge messenden Einheit (91, 91A, 91B) mittels eines verdrahteten Kommunikationssystems über eine Kommunikationsleitung (20, 20A) empfängt.

7. Zahnbehandlungseinheit (10, 10A) nach einem der Ansprüche 1 bis 5,
wobei der Kommunikationsteil (19, 19B) das die Wurzelkanallänge angebende Signal mittels eines drahtlosen Signalkommunikationssystems empfängt.

## Revendications

1. Unité de traitement dentaire (10, 10A) comprenant :
une partie de communication (19, 19B) comprenant un moyen de communication (20A) apte à recevoir un signal qui indique une longueur de canal radiculaire acquise par une unité extérieure de mesure de longueur de canal radiculaire (91, 91A, 91B) avec une fonction de mesure de longueur de canal radiculaire que l'unité de traitement (10, 10A) n'a pas ;
une partie d'affichage (12) apte à afficher la longueur de canal radiculaire ; et
un corps d'unité de traitement (11) utilisé quand un traitement dentaire est dispensé sur la base de la longueur de canal radiculaire.

2. Unité de traitement dentaire (10, 10A) selon la revendication 1, dans laquelle la partie d'affichage (12) affiche également des informations d'entraînement concernant l'unité de traitement elle-même.

3. Unité de traitement dentaire (10, 10A) selon la revendication 1 ou 2, dans laquelle le corps d'unité de traitement (11) a la forme d'une pièce à main avec une partie de préhension (111) qui peut être tenue par un utilisateur.

4. Unité de traitement dentaire (10, 10A) selon la revendication 3, dans laquelle la partie d'affichage (12) est prévue sur la circonférence extérieure de la partie de préhension du corps d'unité de traitement (11).

5. Unité de traitement dentaire (10, 10A) selon l'une quelconque des revendications 1 à 4, dans laquelle la partie d'affichage (12) se compose de l'un au moins des éléments suivants : affichage électroluminescent organique, papier électronique, diode électroluminescente.

6. Unité de traitement dentaire (10, 10A) selon l'une quelconque des revendications 1 à 5, dans laquelle la partie de communication (19, 19B) reçoit le signal indiquant la longueur de canal radiculaire vers et à partir de l'unité extérieure de mesure de longueur de canal radiculaire (91, 91A, 91B) grâce à un système de communication à fil, par l'intermédiaire d'une ligne de communication (20, 20A).

7. Unité de traitement dentaire (10, 10A) selon l'une quelconque des revendications 1 à 5, dans laquelle la partie de communication (19, 19B) reçoit le signal indiquant la longueur de canal radiculaire grâce à un système de communication de signaux sans fil.
